# EUROPÄISCHE PATENTSCHRIFT

(11) **EP 2 023 850 B1**
(45) Veröffentlichungstag und Bekanntmachung des Hinweises auf die Patenterteilung: **03.07.2013**
(21) Anmeldenummer: 07728876.9
(22) Anmeldetag: 08.05.2007
(51) Int. Cl.: A61C 13/00

(54) **ROHLING UND ROHLINGSSORTIMENT MIT VORGEFERTIGTER TEILENDFLÄCHE UND VERFAHREN ZUR HERSTELLUNG VON ZAHNERSATZTEILEN MIT DIESEM ROHLING**
BLANK AND ASSORTMENT OF BLANKS COMPRISING PREFABRICATED PARTIAL END SURFACES AND METHOD FOR PRODUCING PARTIAL DENTURES WITH THIS BLANK
ÉBAUCHE ET ASSORTIMENT D'ÉBAUCHES AVEC FACES TERMINALES PARTIELLES PRÉFABRIQUÉES ET PROCÉDÉ DE FABRICATION D'ÉLÉMENTS DE PROTHÈSE DENTAIRE AVEC CET ÉBAUCHE

(30) Priorität: 08.05.2006 DE 102006021640; 08.05.2006 US 798297 P
(43) Veröffentlichungstag der Anmeldung: 18.02.2009
(73) Patentinhaber: Sirona Dental Systems GmbH, 64625 Bensheim (DE)
(72) Erfinder: ORTH, Ulrich, 64686 Lautertal (DE)
(74) Vertreter: Sommer, Peter
(86) Internationale Anmeldenummer: PCT/EP2007/054423
(87) Internationale Veröffentlichungsnummer: WO 2007/128811

(56) Entgegenhaltungen:
- EP-A1- 1 454 596
- WO-A-01/58378
- WO-A-02/09612
- WO-A2-2005/007007
- DE-U1- 29 621 807

## Beschreibung

### Technisches Gebiet

Die Erfindung betrifft einen Rohling und ein Verfahren zur Herstellung von Zahnersatzteilen, wobei ein 3D-Modell eines anzufertigenden Zahnersatzteils gegeben ist. Der Rohling weist eine in der Geometrie und der Oberflächenbeschaffenheit vorgefertigte Teilendfläche auf, die zumindest mit einer Teilfläche des herzustellenden Zahnersatzteils übereinstimmt.

### Stand der Technik

In der WO 01/58 378 A ist ein Zahnsortiment und Verfahren zur Präparation von Zähnen offenbart. Das Sortiment von fabrikatorisch hergestellten Zähnen weist im zervikalen Bereich größere Außenmaße als ein präparierter Zahn auf. Zur Herstellung wird der vorgefertigte Zahn in einer Vorrichtung befestigt und virtuell mit der Situation des präparierten Zahns korreliert. Eine Innenpassung zum präparierten Zahn wird durch Ausfräsung des Lumens und eine Außenpassung durch Abfräsen bis auf den Präparationsrand hergestellt, um ein passgenaues Anliegen an den präparierten Zahn zu gewährleisten.

Nachteilig ist dabei, dass die vorgefertigten Zähne nur am Präparationsrand im zervikalen Bereich angepasst werden können und somit keine Freiheitsgrade für die individuelle Anpassung der verbleibenden Außenfläche an die anatomische Situation in der Mundhöhle des Patienten gegeben sind.

Das Sortiment aus solchen vorgefertigten Zähnen muss in allen möglichen Varianten bezüglich der Farbgebung und Zahnfunktion vorliegen, um einen farblich und förmlich passenden Zahnersatz zu gewährleisten, wodurch eine hohe Anzahl von vorgefertigten Zähnen im Sortiment erforderlich ist, um die Vielfalt aller möglichen Formen und Farbgebungen von natürlichen Zähnen abzudecken.

Der Zahnersatz wird in einem komplexen Verfahren vollständig vorgefertigt.

Darüber kann es nachteilig sein, dass die Außenflächen des vorgefertigten Zahns im Bereich des Präparationsrands nur ein geringes Übermaß gegenüber der Endform des anzufertigenden Zahnersatzes aufweist. Falls die Kontrolle der Position des verwendeten Bearbeitungswerkzeugs erst bei Kontakt mit dem zu bearbeitenden.Material, beispielsweise durch Feststellung einer Änderung der Leistungsaufnahme eines Elektromotors des Bearbeitungswerkzeugs, erfolgt, ist eine Positionskontrolle erst kurz vor dem Erreichen der Endform des vorgefertigten Zahns möglich.

Oftmals ist es durch die Aufnahme einer Änderung der Leistungsaufnahme des Bearbeitungswerkzeugs auch möglich festzustellen, ob das Bearbeitungswerkzeug zu Bruch gegangen ist und der Elektromotor somit im Leerlauf ist. Da im zervikalen Bereich nur wenig Material abzutragen ist, können bei der Bearbeitung Fehlermeldungen auftreten, obwohl das Bearbeitungswerkzeug nicht gebrochen ist, sondern die auftretenden Reibkräfte mit dem zu bearbeitende Material zu gering sind.

Daher wird im Stand der Technik zwischen dem Halter und dem vorgefertigtem Zahn ein Kalibrierungskörper vorgesehen, dessen Lage zum Zahn bekannt ist. Durch Abfahren des Kalibrierungskörpers ist dann auch die Lage des zu bearbeitenden Zahns bekannt.

In der EP 1 454 596 A1 ist ein Verfahren zur Herstellung von Blenden für Zahnersatz offenbart. Die Blenden weisen dünnwandige Schalen aus Kunststoffmaterial mit einer darauf aufgebrachten Färbungsschicht auf, wobei die Durchsichtigkeit und die Färbung der Blenden dank des mehrschichtigen Aufbaus frei und unabhängig gestaltet werden können. Die Färbungsschicht besteht aus einem Farbstoff zur Farbgebung von transparentem oder transluzentem Zahnersatz. Die Blende wird auf die Oberfläche eines Zahnersatzelement angebracht und weist eine Stärke von 0,2 mm bis 1,5 mm auf.

Nachteilig ist, dass das Anbringen der Blende an einem Zahnersatzelement unter Berücksichtigung des geplanten Transluzenzverlaufs und der Farbgebung des Zahnersatzteils zeitaufwendig ist und einen hohen technischen Aufwand erfordert. Demnach ist es nicht möglich, den Patienten während einer Sitzung mit Zahnersatz zu versorgen.

Aus der DE 198 38 239 A1 ist ein System zur Fertigung von für präparierte Zähne bestimmten Zahnersatzteilen bekannt, bei dem Gruppen von Rohlingen jeweils gleiche Außen- und Innengeometrien aufweisen, wobei die Außengeometrie eines für eine Krone bestimmten Rohlings gleich oder in etwa gleich mit der Außenform des zu ersetzenden Zahns ist.

Nachteilig ist, dass ein sehr großes Rohlingssortiment zur Verfügung stehen muss, um einen zur individuellen Zahnform passenden Rohling mit gleicher Außenform herauszusuchen.

Aus der DE 198 14 762 A1 ist ein Verfahren zur Herstellung von Zahnersatz unter Verwendung von Vorformen, die auf Abdrücken echter menschlicher Zähne verschiedener Größe basieren, bekannt.

Nachteilig ist, dass die einzelnen Vorformen, aus denen der komplette Zahnersatz zusammengesetzt wird, nur an den Zwi schenflächen angepasst werden können, nicht jedoch an den Außenflächen. Folglich muss eine Vielzahl von Vorformen bereit gestellt werden, um die Anforderungen an eine individuelle Zahnersatzform zu erfüllen.

Aus der DE 296 21 807 U1 sind konfektionierte Teilkronen für beschädigte Molaren oder Prämolaren bekannt, die beschädigte oder präparierte Kau- und/oder Mantelflächen dieser Zähne sektor- oder halbkreisförmig bedecken.

Die Teilkronen sind zwar geeignet, einen partiell zerstörten Zahn zu ersetzen, nicht jedoch einen kompletten Zahn.

Aus der US 6 979 496 B2 ist eine Rohlingsdatenbank bekannt, bei der die Außenform der Rohlinge so gewählt wird, dass bei der abschließenden Fräsbearbeitung der Materialabfall minimiert wird.

Nachteilig ist, dass beim vorliegenden Verfahren alle Flächen des Zahnersatzteil mittels Fräsbearbeitung aus dem Rohling herausgefräst werden und dass folglich die ästhetisch relevanten Flächen nachträglich beschichtet werden müssen, um die ästhetischen Anforderungen an einen Zahnersatz zu erfüllen.

Aus der US 5 691 905 A ist ein Verfahren zum Fräsen und Polieren eines Satzes von Zahnformteilen bekannt, bei dem Kunstzähne und Familien von Kunstzähnen verschiedener Größen und/oder Farbgebungen aber gemeinsamer Formen hergestellt werden.

Nachteilig ist, dass die ästhetische relevanten Flächen nach der Bearbeitung nachbearbeitet werden müssen, um die ästhetischen Anforderungen an einen Zahnersatz zu erfüllen.

Die Aufgabe dieser Erfindung besteht daher darin, einen Zahnersatz ohne größeren technischen Aufwand unter Verwendung eines relativ kleinen Rohlingssortiments zu schaffen, der schon bei der ersten Sitzung dauerhaft eingesetzt werden kann und der den ästhetischen Ansprüchen im Vergleich zu einem natürlichen Zahn gleichwohl hinreichend genügt.

### Darstellung der Erfindung

Ein Rohling zur Herstellung eines Zahnersatzteils gemäß der Erfindung weist eine in der Geometrie und den Oberflächeneigenschaften vorgefertigte Teilendfläche des herzustellenden Zahnersatzteils auf. Außerhalb der vorgefertigten ästhetisch relevanten Teilendfläche weist der Rohling die zu bearbeitenden Flächen auf, die ein Übermaß gegenüber der Oberfläche des bereitgestellten Zahnersatzteils aufweisen.

Die vorgefertigte Teilendfläche entspricht bei Schneidezähnen der Labialfläche und vorzugsweise zusätzlich der Inzisalkante bzw. bei Backenzähnen der Bukkalfläche und vorzugsweise zusätzlich der Okklusalfläche.

Die übrigen zu bearbeitenden O-berflächen des Rohlings sind Teil einer geometrischen Grundform , insbesondere eines Kegels, eines Zylinders, eines Würfels, eines Quaders oder einer Pyramide. Solche geometrischen Grundformen sind mit vertretbarem technischen Aufwand herzustellen und erreichen eine hinreichend präzise Festlegung der Oberflächen des Rohlings.

Ein derartiger Rohling bietet genügende Freiheitsgrade für eine Bearbeitung außerhalb der Teilendfläche oder der Flächen am Rand. Auf diese Weise lassen sich mehrere unterschiedliche Zahnersatzteile aus einer Art Rohling herstellten, wodurch sich die Größe des Rohlingssortiments verringern lässt.

Vorteilhafterweise kann die vorgefertigte Teilendfläche höchstens 50% der gesamten Oberfläche des Rohlings ausmachen. Dieser Flächenanteil ist bereits ausreichend, um den Rohling bei einer ausreichenden ästhetischen Qualität individuell passend zur anatomischen Situation im Mundraum des Patienten zu bearbeiten und gleichzeitig die aufwendige Vorfertigung der Teilendfläche gering zu halten.

Das Übermaß kann mindestens 1 mm betragen. Dadurch wird eine rechtzeitige Positionskontrolle eines Bearbeitungswerkzeugs, bei Kontakt mit dem Material, nämlich in einem Abstand zur Oberfläche des geplanten Zahnersatzteils von mindestens 1 mm ermöglicht und darüber hinaus eine Lastkontrolle bei der Bearbeitung.

Vorteilhafterweise kann der Rohling eine Transluzenzschicht aufweisen und die vorgefertigte Teilendfläche auf der Transluzenzschicht angeordnet sein. Dadurch wird ermöglich aus dem Rohling ein Zahnersatzteil herauszuarbeiten, dass zu natürlichen Zähnen ähnliche Transluzenzeingeschaften aufweist.

Vorteilhafterweise kann die Transluzenzschicht eine Dicke zwischen 0,3 mm und 1,5 mm aufweisen und die vorzugsweise poliert ist und in ihrer Form und/oder Transluzenz und/oder Farbgebung unterschiedliche Bereiche aufweist. Die Dicke der Transluzenzschicht verursacht Änderungen der Transluzenzeingenschaften und die Verteilung des Farbstoffs innerhalb der Transluzenzschicht beeinflusst den optischen Farbeindruck.

Vorteilhafterweise kann die vorgefertigte Teilendfläche eine zu den natürlichen Zähnen ähnliche Farbgebung und Transluzenzeigenschaft aufweisen. Die Vielzahl der Rohlinge muss demnach eine hinreichende Anzahl von Farb- und Transluzenzvarianten natürlicher Zähne abdecken.

Für die Anwendung beim erfinderischen Verfahren ist es von Vorteil, wenn die zu bearbeitenden Flächen des Rohlings eine bekannte Grundform aufweisen, denn dadurch können die Rohlinge für die Bestimmung der Rohlingsposition in der Bearbeitungsmaschine herangezogen werden, ohne dass zusätzliche Kalibrierflächen vorhanden sein müssen.

Dabei kann die Lagebeziehung der zu bearbeitenden Oberflächen zueinander, zur Teilendfläche und zu einem Halter des Rohlings bekannt sein. Dadurch ist es möglich durch Messung der Position von mehreren Punkten an der zu bearbeitenden Oberfläche die Position der Teilendfläche bezüglich des Bearbeitungswerkzeugs zu bestimmen.

Weiterhin können die zu bearbeitenden Oberflächen als Referenzflächen zur Bestimmung der Position ausgebildet sein. Dadurch kann vor dem Bearbeitungsvorgang ein Kalibrierungsvorgang an der zu bearbeitenden Fläche zur Bestimmung ihrer Position durchgeführt werden, um die Lage des Rohlings relativ zum Bearbeitungswerkzeug zu bestimmen. Es kann eine Kalibrierungstechnik ausgewählt werden, bei der die Oberflächeneigenschaft der zu untersuchenden Oberfläche beeinträchtigt wird, also insbesondere eine Berührung mit einer Materialabtragung erfolgt.

Der Rohling kann aus Kunststoff oder Keramik, vorzugsweise aus Feldspatkeramik, hergestellt werden. Diese Materialien haben sich in der Zahntechnik bewährt und werden entsprechend den Anforderungen an das herzustellende Zahnersatzteil nach ihren Eigenschaften ausgewählt.

Vorteilhafterweise kann der Rohling aus mindestens zwei Schichten aufgebaut sein, die sich in ihrer Farbgebung voneinander unterscheiden. Geschichtete Rohlinge sind an sich aus dem Stand der Technik bekannt. Die Schichten können aus Kunststoff oder Keramik, vorzugsweise aus Feldspatkeramik, gefertigt sein. Sie können parallel zueinander und entlang der Zahnlängsachse angeordnet sein. Dadurch kann die Farbgebung beispielsweise stufenweise von zervikal nach inzisal in mehreren Schichten dem Farbverlauf natürlicher Zähne angepasst werden. Falls das Material selbst transluzent ist und das Farbmittel im Material verteilt ist, wird das einfallende Licht aus unterschiedlichen Tiefen des gefärbten Materials reflektiert. Der optische Farbtiefeneindruck natürlicher Zähne wird auf diese Weise besser nachgeahmt.

Ein erfindungsgemäßes Rohlingssortiment umfasst mehrere Rohlinge, wobei das Rohlingssortiment mindestens voneinander dem Typ nach gleiche und in den Abmessungen verschiedene Rohlinge wie vorstehend beschrieben umfasst. Zu jedem Zahn mit einer bestimmter Zahnnummer nach dem internationalem Zahnschema liegt demnach ein Rohlingssortiment vor, das Rohlinge mit unterschiedlichen Abmessungen und mit in ihrer Form, Farbgebung und Transluzenzeigenschaften verschiedenen vorgefertigten Teilendflächen umfasst. Aus diesem Rohlingssortiment kann dann ein passender Rohling ausgesucht werden.

Erfindungsgemäß wird außerdem ein Verfahren zur Herstellung eines Zahnersatzteils vorgeschlagen, welches die Bereitstellung eines 3D-Modells eines Zahnersatzteils und die Auswahl eines geeigneten erfindungsgemäßen Rohlings aus einer Mehrzahl von möglichen erfindungsgemäßen Rohlingen umfasst.

Im Rahmen der Bereitstellung des 3D-Modells des Zahnersatzteils ist an dem 3D-Modell des Zahnersatzteils eine ästhetisch relevante Teilkonstruktionsfläche als Teil einer äußeren Oberfläche des Zahnersatzteils festgelegt und aus einer Mehrzahl von erfindungsgemäßen Rohlingen mit einer vorgefertigten ästhetisch relevanten Teilendfläche einer Zahnoberfläche ist ein Rohling bestimmt, dessen Teilendfläche zumindest näherungsweise der festgelegten Teilkonstruktionsfläche entspricht. Das 3D-Modell des Zahnersatzteils wird aus dem ausgewählten Rohling derart herausgearbeitet, dass die vorgefertigte Teilendfläche des Rohlings zumindest in einem zentralen Bereich unbearbeitet bleibt.

Die festgelegte Teilkonstruktionsfläche entspricht einem nach Einbringung des Zahnersatzteils in die Mundhöhle von außerhalb der Mundhöhle sichtbaren Teil der Oberfläche. Aus ästhetischen Gründen sollten sichtbaren Flächen einen zu natürlichen Zähnen ähnlichen optischen Eindruck aufweisen. Dies wird dadurch erreicht, dass eine sichtbare Fläche als Teilkonstruktionsfläche ausgewählt bzw. vorgegebenen wird und aus der vorgefertigten Teilendfläche des Rohlings, die einen zu natürlichen Zähnen ähnlichen optischen Eindruck gewährleistet, herausgearbeitet wird.

Die festgelegte Teilkonstruktionsfläche des geplanten Zahnersatzteils entspricht bei Schneidezähnen der Labialfläche und vorzugsweise zusätzlich der Inzisalkante bzw. bei Backenzähnen der Bukkalfläche und vorzugsweise zusätzlich der Okklusalfläche. Die genannten Flächen sind bei geöffnetem Mund von außen bei normalem Tageslicht sichtbar und sollten aus ästhetischen Gründen in ihrem optischen Eindruck natürlichen Zähnen möglichst entsprechen. Weitere Flächen sind hingegen für den ästhetischen Eindruck von untergeordneter Bedeutung.

In der Zahnmedizin dienen die folgenden Begriffe als Richtungsangaben: okklusal bedeutet zur Kauffläche von Backenzähnen hin, labial bedeutet lippenseitig, bukkal bedeutet backenseitig, mesial bedeutet zur Mitte des Zahnbogens hin, distal bedeutet zum Ende des Zahnbogens hin, inzisal bedeutet zur Schneidekante von Schneidezähnen hin und zervikal bedeutet zum Zahnhals hin. Demnach weist ein Zahn eine seitliche Mesialfläche, eine seitliche Distalfläche, bei Schneidezähnen eine zur Lippe gerichtete Labialfläche und eine Inzisalkante bzw. bei Backenzähnen eine zur Backe zeigende Bukkalfläche und eine Okklusalfläche auf. Die Verbindungsfläche eines Zahnersatzteils ist zum Zahnhals gerichtet und wird als Zervikalfläche bezeichnet.

Die hinteren Backenzähne sind bei Tageslicht von außen nur wenig sichtbar und daher ist für die dort einzusetzenden Zahnersatzteile eine optische Anpassung der Oberfläche nur bedingt erforderlich. Nach der internationalen Zahnschema sind bei den vorderen Zähnen 11-13, 21-23, 31-33, 41-43 die Labialflächen, bei vorderen Backenzähnen 14, 15, 24, 25, 34, 35, 44, 45 die Bukkalflächen und/oder die Okklusalflächen und bei den hinteren Backenzähnen 16-18, 26-28, 36-38, 46-48 die Okklusalflächen als ästhetische Flächen anzusehen, die einer Anpassung des optischen Eindrucks an natürliche Zähne bedürfen.

Der Rohling wird nur im Randbereich der Teilendfläche und außerhalb der Teilendfläche an den zu bearbeitende Flächen bearbeitet. Dadurch wird zumindest der zentrale Bereich der Teilendfläche ausgespart und weist demnach ästhetische Oberflächeneigenschaften der vorgefertigten Teilendfläche auf.

Das 3D-Modell des Zahnersatzteils wird beispielsweise durch Konstruktion mittels eines digitalen Zahnersatzplanungssystems passend zur Präparationsstelle bereitgestellt und in einem Datenspeicher abgelegt, um im erfindungsgemäßen Verfahren verwendet zu werden. Die ästhetisch relevante Teilkonstruktionsfläche ist eine Teilfläche des vorgegebenen konstruierten 3D-Modells, die anhand ihrer Lage und Form ausgewählt ist. Beispielsweise kann als ästhetisch relevante Teilkonstruktionsfläche eine Fläche ausgewählt werden, die später bei offenem Mund von außerhalb der Mundhöhle gesehen sichtbar wäre und die aus ästhetischen Gründen einen zu den natürlichen Zähnen ähnlichen optischen Eindruck aufweisen soll.

Die Auswahl des Rohlings mit einer zumindest näherungsweise der Teilkonstruktionsfläche entsprechenden vorgefertigten Teilendfläche aus einer Mehrzahl von Rohlingen kann vorzugsweise in Form einer computergestützten Auswahl aus einer Mehrzahl von in einem Datenspeicher abgelegten 3D-Modellen von Rohlingen erfolgen. Die übrige Oberfläche des Rohlings ist demnach nicht vorgefertigt und wird auf die Endform des geplanten Zahnersatzteils hin bearbeitet. Die Bearbeitung des ausgewählten Rohlings kann beispielsweise durch eine computergesteuerte Bearbeitungsmaschine nach einer berechneten Bearbeitungsroutine erfolgen.

Nach dem erfindungsgemäßen Verfahren kann demnach aus einem erfindungsgemäßen Rohling mit einer bestimmten Teilendfläche eine Vielzahl von unterschiedlichen Zahnersatzteilen mit einerseits zur Teilendfläche ähnlichen Teilkonstruktionsflächen und andererseits mit unterschiedlicher Außenform außerhalb der Teilendfläche gefertigt werden. Beim Herstellungsverfahren des Rohlings muss im Gegensatz zur vollständigen Vorfertigung entsprechend dem geplanten Zahnersatzteil nur eine bestimmte Teilendfläche ästhetisch, beispielsweise in ihrer Form, Farbgebung und Transluzenz, vorgefertigt wird. Dies vereinfacht das Herstellungsverfahren.

Die Fertigung des Zahnersatzteils aus dem ausgewählten Rohling kann getrennt von der Planung des Zahnersatzteils erfolgen, beispielsweise in einem Zahntechnikerlabor.

Die zumindest näherungsweise zu der Teilkonstruktionsfläche ähnliche Teilendfläche tritt an die Stelle der Teilkonstruktionsfläche. Es wird also der ästhetische Eindruck der vorgefertigten Teilendfläche möglichst unverändert gelassen, dafür aber die äußere Form des 3D-Modells des Zahnersatzteils im Bereich der ästhetisch relevanten Teilkonstruktionsfläche durch zumindest einen ähnlichen Teil der Teilendfläche ersetzt. Das gefertigte Zahnersatzteil kann also im Bereich der Teilkonstruktionsfläche geringfügig vom vorgegebenen digitalen 3D-Modell des anzufertigenden Zahnersatzteils abweichen, weil beim Bearbeiten der betreffende Bereich der vorgefertigten Teilendfläche ausgespart wird. Wenn es sich bei der Teilkonstruktionsfläche beispielsweise um eine sichtbare Fläche handelt, hat die geringe Abweichung keine Auswirkung auf die Passgenauigkeit zu den Nachbarzähnen.

Vorteilhafterweise wird im Rahmen der Bereitstellung des 3D-Modells des Zahnersatzteils ein zu der Präparationsstelle passendes 3D-Modell eines Zahns aus einer Zahndatenbank mit einer Mehrzahl von 3D-Modellen eines Zahns mit mindestens einer festgelegten ästhetisch relevanten Teilendfläche ausgewählt. Zu jedem 3D-Modell eines Zahns aus der Zahndatenbank ist ein 3D-Modell eines Rohlings mit einer mit der Teilendfläche des Zahns übereinstimmenden Teilendfläche in einer Rohlingsdatenbank vorhanden. Das ausgewählte 3D-Modell des Zahns wird zur Anpassung an die individuelle Zahnsituation der Präparationsstelle verändert, wobei die festgelegte Teilendfläche zumindest in einem zentralen Bereich unverändert bleibt. Weiterhin ist ein zu der Rohlingsdatenbank passendes Rohlingssortiment vorhanden und das 3D-Modell des Zahnersatzteils wird aus dem dem 3D-Modell des Zahns entsprechenden Rohling herausgearbeitet.

Die 3D-Modelle der Zähne aus der Zahndatenbank und die entsprechenden 3D-Modelle der Rohlinge aus der Rohlingsdatenbank, die in einem Rohlingssortiment vorliegen, weisen übereinstimmende Teilendflächen mit vorgegebenen Grenzen auf. Das 3D-Modell des Zahns wird geringfügig verändert, ohne dabei jedoch zumindest den zentralen Bereich der Teilendfläche zu verändern, und es wird ein individuell angepasstes 3D-Modell des Zahnersatzteils erzeugt. Anschließend wird aus dem entsprechenden Rohling des Rohlingssortiments das Zahnersatzteil herausgearbeitet. Die Lagebeziehung zwischen der Teilkonstruktionsfläche und der Teilendfläche ist inhärent gegeben und muss nicht zusätzlich bestimmt werden, denn die Teilkonstruktionsfläche wird aus der Teilendfläche des 3D-Modells des Zahns der Zahndatenbank erzeugt und stimmt zumindest im zentralen Bereich mit der Teilkonstruktionsfläche überein. Sobald die Lage des Rohlings in der Bearbeitungsmaschine bekannt ist, kann auch der Bearbeitungsplan erstellt werden, weil die Lagebeziehung bekannt ist.

Eine Teilkonstruktionsfläche an dem 3D-Modell des bereitgestellten Zahnersatzteils um nicht anhand von 3D-Modellen von Zähnen aus einer Zahndatenbank bestimmt werden, sondern kann entweder interaktiv durch den Benutzer unter Verwendung von Eingabemitteln oder automatisch durch einen Computer anhand von vorgegebenen Parametern festgelegt werden. Beispielsweise kann der Benutzer in einer Softwareapplikation über Eingabemittel wie Tastatur und Maus die Teilkonstruktionsfläche markieren, indem er beispielsweise die Grenzlinie zieht oder vordefinierte Zellen puzzleartig auswählt. Die Teilkonstruktionsfläche kann auch computergestützt automatisch anhand von vorgegebenen Parametern ausgewählt werden. Es kann beispielsweise der Lichteinfall und die Sichtbarkeit der Zahnoberflächen computergestützt simuliert werden und die als sichtbar bestimmte Teilfläche automatisch als die Teilkonstruktionsfläche definiert werden. Die Teilkonstruktionsfläche kann also auch von einem Teilbereich der Teilendfläche geringfügig abweichen, weil das 3D-Modell ohne Berücksichtigung einer Teilendflächen aufweisenden Zahndatenbank bereitgestellt wird. Die Auswahl eines 3D-Modells eines Rohlings aus einer Mehrzahl in beispielsweise einem Datenspeicher abgelegter 3D-Modelle von Rohlingen mit mindestens einer vorgefertigten Teilendfläche einer Zahnoberfläche kann automatisch durch den Computer erfolgen. Die Abweichungen zwischen der festgelegten Teilkonstruktionsfläche am 3D-Modell des Zahnersatzteils und der vorgefertigten Teilendfläche werden dabei minimiert. Dadurch wird ein Rohling ausgesucht, dessen Teilendfläche der Teilkonstruktionsfläche am besten entspricht.

Das 3D-Modell des Zahnersatzteils kann aus dem ausgewählten Rohling derart herausgearbeitet werden, dass im angefertigten Zahnersatzteil zumindest ein Teilbereich der vorgefertigten Teilendfläche des Rohlings an die Stelle der ausgewählten Teilkonstruktionsfläche des 3D-Modells des Zahnersatzteils tritt. Dadurch bleibt die vorgefertigte Teilendfläche in dem zur Teilkonstruktionsfläche ähnlichen Bereich unbearbeitet, wobei jedoch dort das gefertigte Zahnersatzteil vom bereitgestellten 3D-Modell des Zahnersatzteil geringfügig abweicht. Bei den ästhetisch relevanten Flächen wie den sichtbaren Flächen führt dies jedoch nicht zu Passungenauigkeiten des Zahnersatzteils.

Die Minimierung der Abweichungen kann so erfolgen, dass sich die Abweichungen hauptsächlich im Randbereich der Teilkonstruktionsfläche befinden. Die Teilkonstruktionsfläche wird computergestützt mit verschiedenen Teilbereichen der Teilendfläche verglichen und der Rohling mit geringsten Abweichungen ausgewählt. Die Abweichungen sollten sich im Randbereich befinden, weil der optische Eindruck im Randbereich weniger wesentlich ist.

Weiterhin kann eine Lagebeziehung zwischen der festgelegten Teilkonstruktionsfläche des 3D-Modells des Zahnersatzteils und der vorgefertigten Teilendfläche des Rohlings festgestellt werden, um die Teilkonstruktionsfläche des 3D-Modells des Zahnersatzteils mit dem ähnlichen Teilbereich der vorgefertigten Teilendfläche im 3D-Modell in Übereinstimmung zu bringen. Dies kann durch Darstellung und eine Sichtkontrolle des 3D-Modells vom Zahnersatzteil innnerhalb des 3D-Modell vom ausgewählten Rohling erfolgen. Die Positionierung des 3D-Modells des Zahnersatzteils zur Übereinstimmung kann automatisch computergestützt oder durch den Benutzer durchgeführt werden. Das 3D-Modell vom Zahnersatzteil kann beispielsweise innerhalb des 3D-Modells des Rohlings graphisch an einem Anzeigemittel dargestellt und durch den Benutzer mittels Eingabemitteln positioniert und somit die Lagebeziehung ermittelt werden.

Anhand der Lagebeziehung kann ein Bearbeitungsplan für den ausgewählten Rohling bestimmt werden. Der Bearbeitungsplan berücksichtigt dann sowohl die Flächen, die nicht bearbeitet werden dürfen als auch die Flächen, die noch bearbeitet werden müssen.

Vorteilhafterweise kann der Rohling eine Transluzenzschicht aufweisen und die vorgefertigte Teilendfläche auf der Transluzenzschicht angeordnet sein. Dadurch kann die Transluzenzeigenschaft von natürlichen Zähnen nachgebildet werden und der ästhetische Eindruck verbessert werden.

Die vorgefertigte Teilendfläche des Rohlings kann die Teilkonstruktionsfläche des Zahnersatzteils umfassen und die auf der Transluzenzschicht angeordnete Teilendfläche bis auf die Teilkonstruktionsfläche bearbeitet werden. Anschließend kann der bearbeitete Rand der Transluzenzschicht poliert werden. Dadurch wird die durch den Schleifvorgang verminderte Transluzenzeigenschaft an den Rändern der Transluzenzschicht wiederhergestellt.

Die Teilkonstruktionsfläche des Zahnersatzteils kann die vorgefertigte Teilendfläche des Rohlings umfassen. Der übriggebliebene Randbereich zwischen der vorgefertigten Teilendfläche und der Teilkonstruktionsfläche, der zunächst keine Transluzenzschicht aufweist, kann dann nachträglich mit einer Transluzenzschicht verkleidet und poliert werden. Dadurch wird die gewünschte Transluzenzeigenschaft der gesamten Teilkonstruktionsfläche auch im Randbereich gewährleistet.

Vorteilhafterweise können die zu bearbeitenden Flächen außerhalb der Teilendfläche des Rohlings ein Übermaß zur Oberfläche des anzufertigenden Zahnersatzteils von mindestens 1 mm aufweisen.

Die Kontrolle eines Bearbeitungswerkzeugs, beispielsweise eines Schleifers, erfolgt oft durch Kontakt mit dem zu bearbeitenden Material. Dadurch kann also die Positionskontrolle mindestens 1 mm vor dem Erreichen der Oberfläche des geplanten Zahnersatzteils durchgeführt und eine präzise Bearbeitung ermöglicht werden. Darüber hinaus lässt sich das Bearbeitungswerkzeug während der Bearbeitung mit hinreichend großer elektrischen Last im Rohling bewegen, so dass eine lastabhängige Steuerung oder Kontrolle möglich ist.

Vorteilhafterweise kann die festgelegte Teilkonstruktionsfläche höchstens 50% der gesamten äußeren Oberfläche des 3D-Modells des Zahnersatzteils ausmachen. Dadurch bleibt der Herstellungsaufwand des Rohlings in Grenzen und es verbleiben genügend Freiheitsgrade, um den Rohling passgenau zur individuellen anatomischen Situation im Mundraum des Patienten zu bearbeiten, wobei zumindest der zentrale Bereich der vorgefertigte Teilendfläche ausgespart wird.

Vorteilhafterweise kann der Rohling aus einer Vielzahl von Rohlingen ausgewählt werden, die in ihrer Form und/oder Farbgebung und/oder Transluzenzeigenschaften abweichende vorgefertigte Teilendflächen aufweisen. Die Transluzenzschicht weist eine zu natürlichen Zähnen ähnliche Farbgebung und Transluzenzeigenschaft auf. Daher muss eine Vielzahl von Rohlingen mit Transluzenzschichten bereitgestellt werden, die die Farbgebungs- und Transluzenzvielfalt verschiedener natürlicher Zähne abdeckt.

Kurzbeschreibung der Zeichnungen Ausführungsbeispiele der Erfindung sind in der Zeichnung dargestellt. Es zeigt die
- Fig. 1A: eine Draufsicht eines Rohlings für einen Schneidezahn;
- Fig. 1B: eine Schnittdarstellung des Rohlings aus Fig.1A entlang einer Schnittlinie AA aus Fig. 1A;
- Fig. 2A: eine Draufsicht eines fast fertigen Zahnersatzteils, das aus dem Rohling in Fig. 1A, 1B herausgearbeitet wurde;
- Fig. 2B: eine Schnittdarstellung des bearbeiteten Rohlings aus Fig.2A entlang einer Schnittlinie BB;
- Fig. 3A: eine bukkale Seitenansicht eines Rohlings für einen Backenzahn;
- Fig. 3B: eine Draufsicht des Rohlings aus Fig. 3A;
- Fig. 4A: eine bukkale Seitenansicht eines fast fertigen Zahnersatzteils, das aus dem Rohling in Fig. 3A, 3B herausgearbeitet wurde;
- Fig. 4B: eine Draufsicht des bearbeiteten Rohlings aus Fig. 4A;
- Fig. 5A: eine Draufsicht eines Rohlings für einen Schneidezahn mit einer über eine Teilendfläche hinausragenden Teilkonstruktionsfläche;
- Fig. 5B: eine Schnittdarstellung des Rohlings aus Fig.5A entlang einer Schnittlinie CC;
- Fig. 6A: eine Draufsicht eines fast fertigen Zahnersatzteils, das aus dem Rohling in Fig. 5A herausgearbeitet wurde;
- Fig. 6B: eine Schnittdarstellung des bearbeiteten Rohlings aus Fig.6A entlang Schnittlinie DD;
- Fig. 7: eine Vorrichtung zur Durchführung des Verfahrens;
- Fig. 8: 3D-Modelle von Zähnen aus einer Zahndatenbank, die mittels einer Anzeigeeinheit angezeigt sind;
- Fig. 9: 3D-Modelle von Rohlingen aus einer Rohlingsdatenbank, die mittels einer Anzeigeeinheit angezeigt sind und zu der Zahndatenbank aus Fig. 8 entsprechen;
- Fig. 10: Rohlinge aus einem Rohlingssortiment zu den 3D-Modellen von Rohlingen aus Fig. 9.

### Ausführungsbeispiele der Erfindung

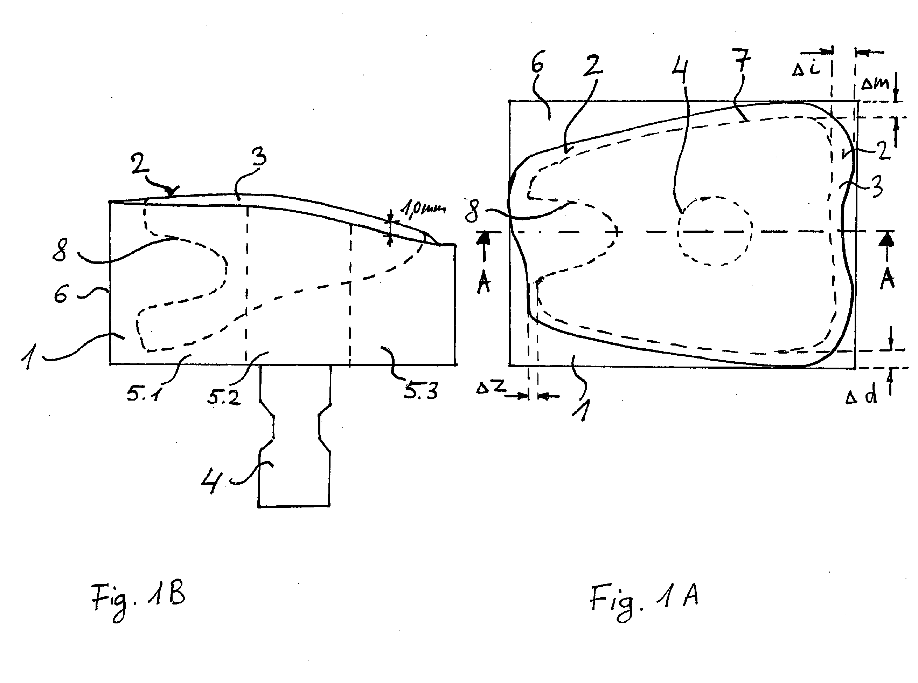

Die Fig. 1A zeigt eine Draufsicht eines Rohlings 1 zur Herstellung eines Zahnersatzteils für einen Schneidezahn und die Fig. 1B zeigt eine Schnittdarstellung entlang der Schnittlinie AA. Der Rohling 1 weist eine vorgefertigte Teilendfläche 2 auf, die auf einer Transluzenzschicht 3 angeordnet ist. Die vorgefertigte Teilendfläche 2 entspricht in ihrer Form einem unteren rechten Schneidezahn mit der Zahnnummer 41 nach dem internationalem Zahnschema. Der Rohling 2 umfasst einen Halter 4, um den Rohling 1 in eine nicht dargestellte Bearbeitungsmaschine einzuspannen.

Die vorgefertigte Teilendfläche 2 ist in ihrer Form einer Labialfläche eines Schneidezahns nachgebildet. Die Transluzenzschicht 3 weist eine maximale Dicke von 1 mm am inzisalen Ende der vorgefertigten Fläche auf und führt dazu, dass die auf der Transluzenzschicht 3 angeordnete vorgefertigte Teilendfläche 2 ähnliche Transluzenzeigenschaften wie natürlichen Schneidezähne aufweist.

Der Rohling 1 kann aus Feldspatkeramik gefertigt sein und aus drei Schichten 5.1, 5.2 und 5.3 mit unterschiedlichen Farbeigenschaften bestreben, wobei die Schichten 5.1 bis 5.3 so ausgebildet und angeordnet sein können, dass der Farbverlauf natürlicher Schneidezähne möglichst nachgeahmt wird. Geschichtete Rohlinge mit unterschiedlichen Farben sind aus dem Stand der Technik bekannt. Die zu bearbeitenden Flächen 6 sind Teil eines rechtwinkligen Quaders, können aber auch andere geometrische Grundformen aufweisen, insbesondere Kegel, Zylinder und Pyramide.

Die Lagebeziehung der zu bearbeitenden Flächen 6 zum Halter 4 und zur Teilendfläche 2 ist bekannt. Daher kann erfindungsgemäß durch Messung der Position an verschiedenen Punkten der Flächen 6 die Position des Rohlings und somit der Teilendfläche 2 relativ zum Bearbeitungswerkzeug bestimmt werden.

Innerhalb der Teilendfläche 2 ist eine Teilkonstruktionsfläche 7 eines anzufertigenden Zahnersatzteils dargestellt, welches als digitales 3D-Modell vorliegt. Diese Teilkonstruktionsfläche 7 weist am inzisalen Rand einen Abstand Δi, am zervikalen Rand einen Abstand Δz, am mesialen Rand einen Abstand Δm und am distalen Rand einen Abstand Δd auf. An der zervikalen Seite weist das geplante Zahnersatzteil ein Lumen 8 zur Verbindung mit einem nicht dargestellten präparierten Zahnstumpf auf. Der Rohling 1 kann so aus einem Sortiment ausgewählt sein, dass die Teilkonstruktionsfläche 7 mit dem inneren Bereich der vorgefertigten Teilendfläche 2 näherungsweise übereinstimmt.

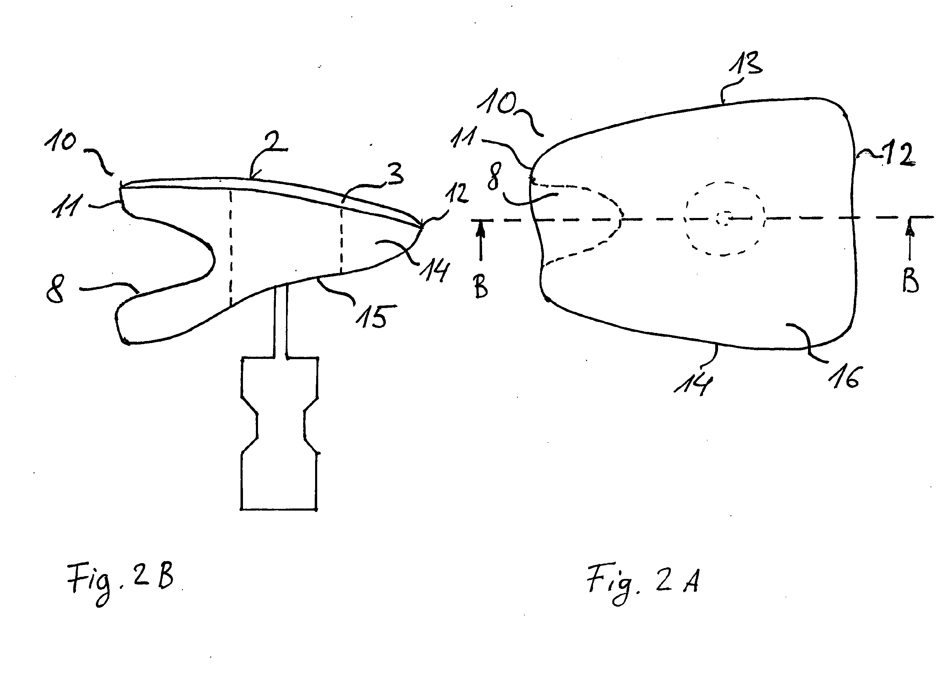

Die Fig. 2A zeigt eine Draufsicht von labial eines Zahnersatzteils 10 für einen Schneidezahn, das aus dem in Fig. 1A dargestellten Rohling herausgearbeitet wurde und die Fig. 2B zeigt eine Schnittdarstellung des Zahnersatzteils 10 entlang der Schnittlinie BB aus Fig. 2A. Aus dem Rohling in Fig. 1A, 1B wurden eine Zervikalfläche 11 mit einem Lumen 8, eine Inzisalkante 12, eine Mesialfläche 13, eine Distalfläche 14 und eine Lingualfläche 15 automatisch herausgearbeitet, wobei Teilendfläche 2 im Umfang der Teilkonstruktionsfläche 7 aus Fig. 1A, 1B , die einer Labialfläche 16 entspricht, nicht bearbeitet wurde.

Am Rand der Labialfläche 16 wurde die vorgefertigte Teilendfläche 2 mit der transluzenten Schicht 3 während des Schleifvorgangs bearbeitet und kann anschließend poliert werden, um einen gleichmäßigen optischen Übergang zwischen der nicht beschliffenen Labialfläche 16 und den übrigen beschliffenen Flächen 12 bis 24 herzustellen.

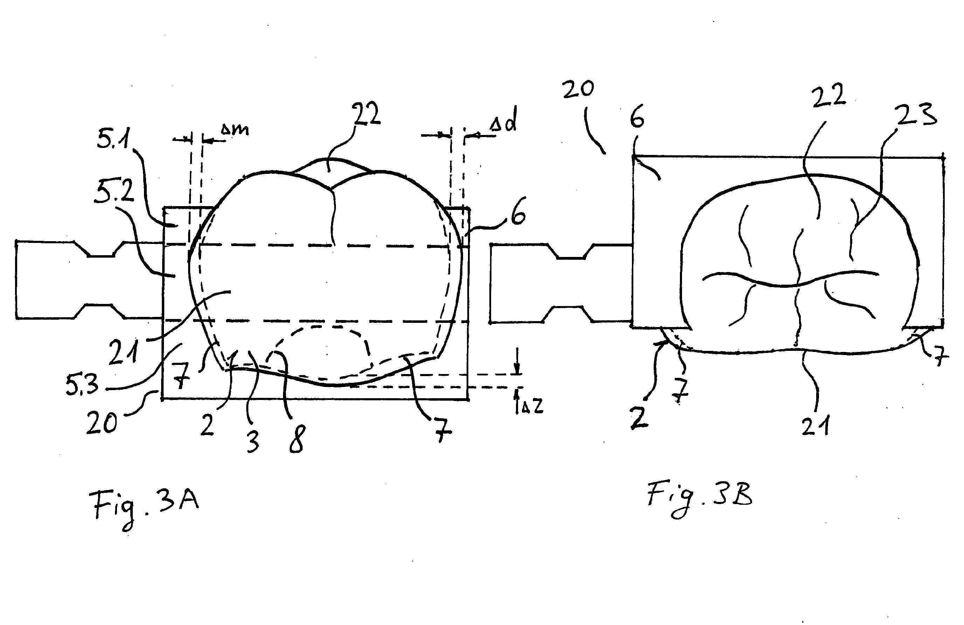

Die Fig. 3A zeigt eine Seitenansicht von bukkal eines Rohlings 20 zur Herstellung eines Zahnersatzteils für einen Backenzahn und Fig. 3B zeigt eine okklusale Draufsicht des Rohlings 20. Der Rohling 20 beinhaltet eine vorgefertigte Teilendfläche 2 auf einer Transluzenzschicht 3. Die vorgefertigte Teilendfläche 2 ist in ihrer Form einer Bukkalfläche 21 und einer Okklusalfläche 22 eines unteren rechten Backenzahns mit der Zahnnummer 45 nach dem internationalem Zahnschema nachgebildet. Die Transluzenzschicht 3 kann eine maximale Dicke von 1,5 mm aufweisen. Der Rohling 20 kann aus Feldspatkeramik gefertigt sein und aus drei Schichten 5.1, 5.2 und 5.3 mit unterschiedlichen Farbeigenschaften bestehen, so dass der Farbverlauf natürlicher Backenzähne möglichst nachgeahmt wird. Die zu bearbeiteten Flächen 6 bilden hier wider einen rechtwinkligen Quader.

Innerhalb der Teilendfläche 2 ist eine Teilkonstruktionsfläche 7 des geplanten Zahnersatzteils dargestellt, wobei die Okklusalfläche 22 und der Hauptteil der Bukkalfläche 21 unverändert bleibt. Diese Teilkonstruktionsfläche 7 weist am zervikalen Rand einen Abstand Δz, am mesialen Rand einen Abstand Δm und am distalen Rand einen Abstand Δd zum Rand der Teilendfläche 2 auf. An der zervikalen Seite weist das geplante Zahnersatzteil ein Lumen 8 zur Verbindung mit einem nicht dargestellten präparierten Zahnstumpf auf. Die vorgefertigte Okklusalfläche 22 umfasst Fissuren 23, die in ihrem optischen Eindruck einem natürlichen Backenzahn ähneln.

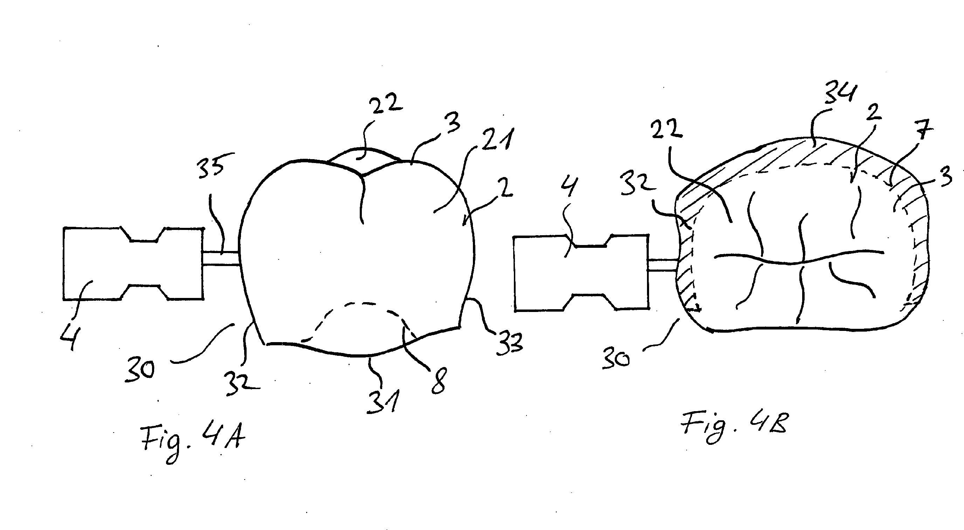

Die Fig. 4A zeigt eine Seitenansicht von bukkal eines Zahnersatzteils 30, das aus dem Rohling in Fig. 3A herausgearbeitet wurde und Fig. 4B zeigt eine okklusale Draufsicht des Zahnersatzteils 30.

Während des Bearbeitungsvorgangs wurde eine Zervikalfläche 31 mit einem Lumen 8 im inneren des Zahnersatzteils 30, das gestrichelt dargestellt ist, eine Mesialfläche 32, eine Distalfläche 33 und eine Lingualfläche 34, die schraffiert dargestellt sind, aus dem Rohling in Fig.3A herausgearbeitet, wobei die Teilendfläche 2 im Umfang der Teilkonstruktionsfläche 7, umfassend die Bukkalfläche 21 und die Okklusalfläche 22, nicht bearbeitet wurde und ein zylinderförmiger Verbindungssteg 35, der das Zahnersatzteil 30 mit dem Halter 4 verbindet, stehen gelassen wurde. Am Rand der Teilkonstruktionsfläche 7 kann die bearbeitete Transluzenzschicht 3 anschließend poliert werden, um die gewünschte Transluzenzeingenschaft im Randbereich der Teilkonstruktionsfläche 7 herzustellen.

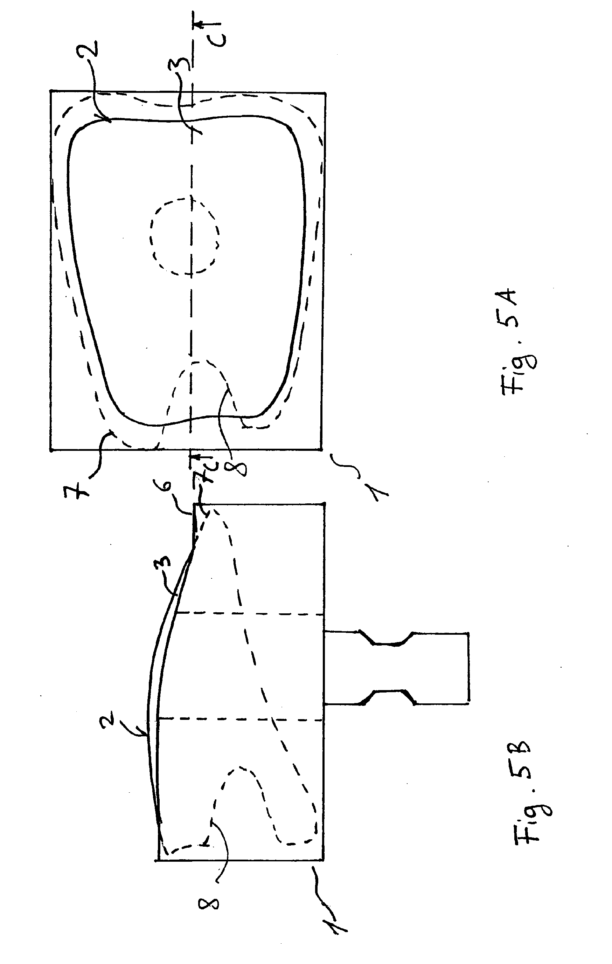

Die Fig. 5A zeigt eine Draufsicht eines Rohlings 1 zur Herstellung eines Zahnersatzes für einen Schneidezahn mit einer über die Teilendfläche 2 hinausragenden Teilkonstruktionsfläche 7 und Fig. 5B zeigt eine Schnittdarstellung des Rohlings 1. Die auf der Transluzenzschicht 3 angeordnete vorgefertigte Teilendfläche 2 ist also kleiner als in Fig. 1 und die geplante Teilkonstruktionsfläche 7 des anzufertigenden Zahnersatzteils umschließt die Teilendfläche 2. Beim Bearbeitungsvorgang wird der Rohling 1 bis auf die Teilendfläche 2 bearbeitet.

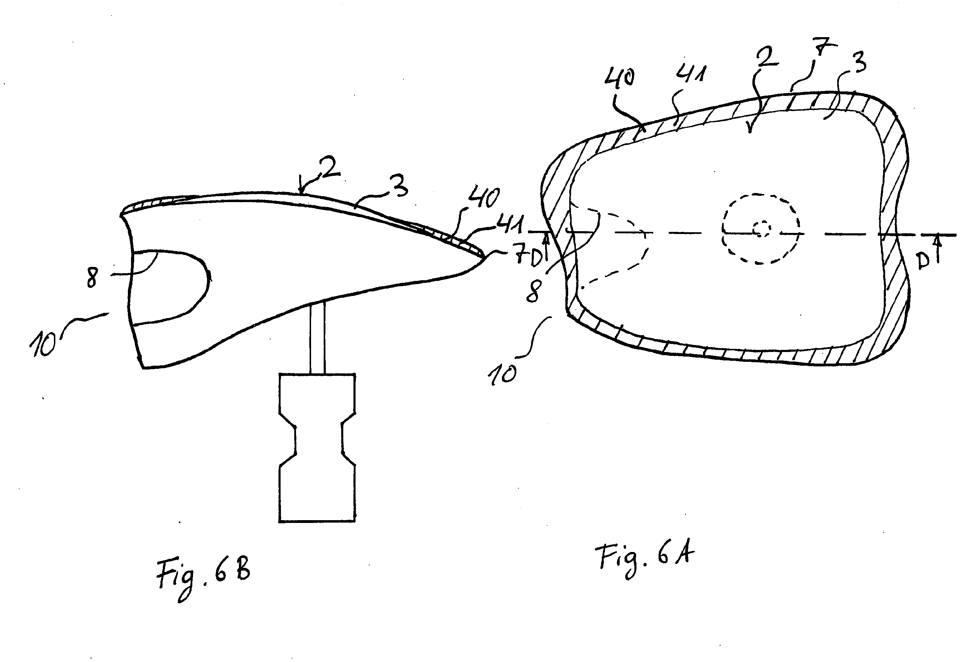

Die Fig. 6A zeigt eine Draufsicht eines Zahnersatzteils 10, das aus dem Rohling in Fig. 5A herausgeschliffen wurde und die Fig. 6B eine Schnittdarstellung des Rohlings aus Fig.6A entlang einer Schnittlinie DD. Beim Schleifvorgang wurden der Randbereich 40 zwischen der vorgefertigten Teilendfläche 2 und der Teilkonstruktionsfläche 7, der in Fig. 6A schraffiert dargestellt ist, beschliffen und weist folglich keine Transluzenzschicht mehr auf. Zur Wiederherstellung der Transluzenzeingeschaft im Randbereich 40 wird deshalb der Randbereich 40 anschließend mit einer Transluzenzschicht 41, die in Fig. 6B schraffiert dargestellt ist, verkleidet.

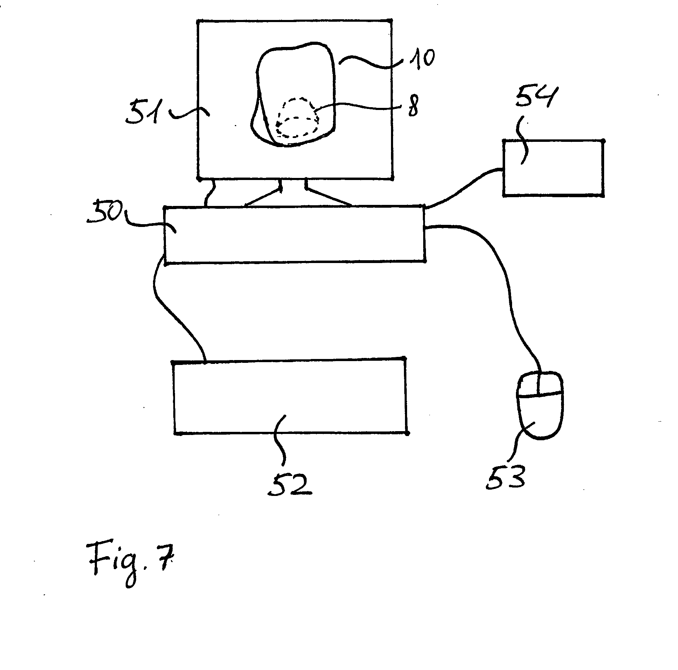

Die Fig. 7 zeigt einen Computer 50, der eine Anzeigeeinheit 51, eine Computertastatur 52, eine Computermaus 53 und einen Datenspeicher 54 umfasst. Die Anzeigeeinheit 51 zeigt ein 3D-Modell eines anzufertigenden Zahnersatzteils 10 für einen Schneidezahn mit einem Lumen 8. Der Computer 50 kann zur Datenverarbeitung bei der Konstruktion des 3D-Modells des anzufertigenden Zahnersatzteils und bei der Planung der Fertigung des Zahnersatzteils 10, 30 (Fig. 2, Fig. 4, Fig. 6) dienen. Dabei wird die Teilkonstruktionsfläche 7 (Fig. 2, Fig. 4, Fig. 6) des geplanten Zahnersatzteils wahlweise entweder manuell durch den Benutzer unter Verwendung der Eingabemittel 3 und 4 oder automatisch unter Verwendung des Computers 1 ausgewählt. Aus der Position des Zahnersatzteils im Mundraum des Patienten kann durch den Computer 1 gestützt unter Anwendung von Computeralgorithmen der Lichteinfall durch die Mundöffnung simuliert und die von außen sichtbare Fläche automatisch bestimmt und als Teilkonstruktionsfläche 7 (Fig. 2, Fig. 4, Fig. 6) des Zahnersatzteils festgelegt werden. Eine weitere Ausführungsmöglichkeit ist, dass die Teilkonstruktionsfläche 7 (Fig. 2, Fig. 4, Fig. 6) anhand von im Datenspeicher 5 abgelegten Bibliothekdaten anderer bereits geplanter 3D-Modelle mit ausgewählten Teilkonstruktionsflächen von Zahnersatzteilen für den entsprechenden Zahn mit bestimmter Zahnnummer automatisch bestimmt wird.

Ein Rohling 1, 20 mit einer vorgefertigten Teilendfläche 2 (Fig. 1, Fig. 3, Fig. 5) wird automatisch durch den Computer 50 oder manuell unter Anwendung der Eingabemittel 52 und 53 durch den Benutzer ausgewählt, so dass die Teilendfläche 2 in ihrer Form zumindest näherungsweise der ausgewählten Teilkonstruktionsfläche 7 des geplanten Zahnersatzteils entspricht. Das 3D-Modell des geplanten Zahnersatzteils kann innerhalb des 3D-Modells des ausgewählten Rohlings 1, 20 an der Anzeigeeinheit 2 dargestellt und so positioniert werden, dass die Teilkonstruktionsfläche 7 mit der Teilendfläche 2 möglichst übereinstimmt. Weiterhin kann eine Lagebeziehung bestimmt werden, um die vorgegebene Teilendfläche in das 3D-Modell einzupassen.

Anschließend wird automatisch eine Bearbeitungsplan berechnet, um das geplante Zahnersatzteil 10, 30 (Fig. 2, Fig. 4, Fig. 6) aus dem ausgewählten Rohling 1, 10 herauszuarbeiten.

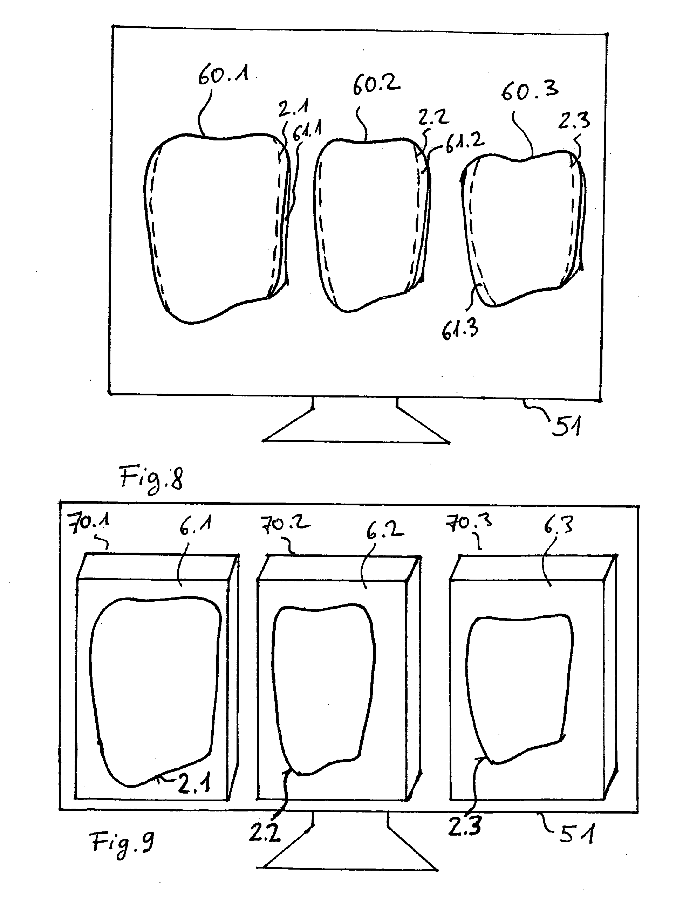

Die Fig. 8 zeigt drei 3D-Modelle 60.1, 60.2 und 60.3 von Zähnen einer Zahndatenbank, die mittels der Anzeigeeinheit 51 angezeigt werden, wobei die Zahndatenbank im Datenspeicher 54 abgelegt ist. Die in ihren Abmessungen und Form unterschiedliche 3D-Modelle 60.1, 60.2 und 60.3 von Zähnen stellen mögliche Varianten eines Zahns mit der Zahnnummer 41 nach dem internationalem Zahnschema dar. Die 3D-Modelle 60.1, 60.2 und 60.3 weisen definierte ästhetisch relevante Teilendflächen 2.1, 2.2 und 2.3 auf, die sich in geplanten Eigenschaften wie der Form, Farbgebung und Transluzenzeigenschaft unterscheiden. Die außerhalb der Teilendflächen 2.1, 2.2 und 2.3 liegenden Restflächen 61.1, 61.2 und 61.3 unterscheiden sich in ihrer Form, die der Form natürlicher Zähne ähnelt. Beim erfinderischen Verfahren wird ein zu der Präparationsstelle passendes 3D-Modell aus den 3D-Modellen 60.1 bis 60.3 der Zahndatenbank mit festgelegten Teilendflächen 2.1 bis 2.3 ausgewählt. Anschließend wird das ausgewählte 3D-Modell zur Anpassung an die individuelle Zahnsituation der Präparationsstelle so verändert, dass die festgelegten Teilendfläche 2.1 bis 2.3 zumindest in einem zentralen Bereich unverändert bleibt. Dadurch entsteht ein individuelles, an die Präparationsstelle angepasstes 3D-Modell des herzustellenden Zahnersatzteils mit einer Teilkonstruktionsfläche, die einer Teilendfläche eines Rohlings zumindest näherungsweise entspricht.

Die Fig. 9 zeigt drei 3D-Modelle von Rohlingen 70.1, 70.2 und 70.3 aus einer Rohlingsdatenbank, die im Datenspeicher 54 aus der Fig. 7 abgelegt sind und auf dem Anzeigemittel 51 angezeigt werden. Die 3D-Modelle 70.1, 70.2 und 70.3 aus der Rohlingsdatenbank entsprechen 3D-Modellen 60.1, 60.2 und 60.3 aus der Zahndatenbank und weisen übereinstimmende Teilendflächen 2.1, 2.2 und 2.3 auf. Die Lagebeziehung der zu bearbeitenden Oberflächen 6.1, 6.2 und 6.3 zueinander, zu den Teilendflächen 2.1, 2.2 und 2.3 ist bekannt. Bei Kenntnis der Oberflächendaten eines 3D-Modells des Zahnersatzteils und der Oberflächendaten des 3D-Modells des Rohlings, aus dem das Zahnersatzteils herauszuarbeiten ist, ist es möglich einen Bearbeitungsplan für das Bearbeitungswerkzeug zu erzeugen.

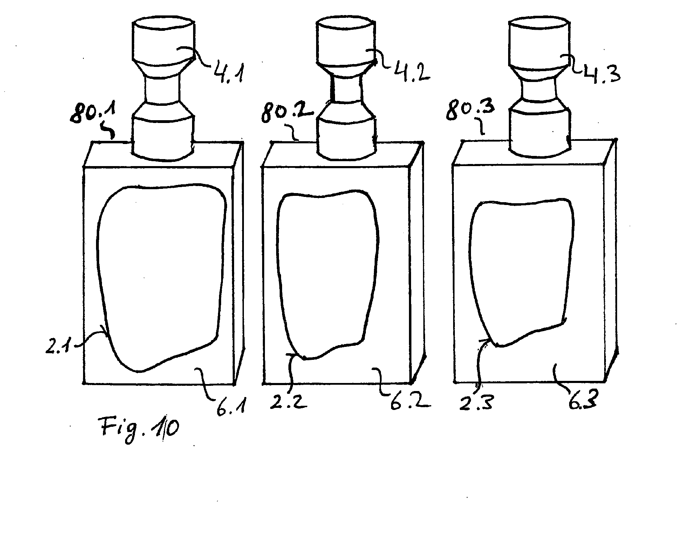

Die Fig. 10 zeigt drei Rohlinge 80.1, 80.2 und 80.3 des Rohlingssortiment, die nach den 3D-Modellen 70.1, 70.2 und 70.3 aus der Rohlingsdatenbank gefertigt wurden. Die Rohlinge 80.1 bis 80.3 weisen zu bearbeitende Oberflächen 6.1, 6.2 und 6.3 und Halter 4.1, 4.2 und 4.2 zum Einspannen in die Bearbeitungsmaschine auf. Beim erfinderische Verfahren wird das Zahnersatzteil aus einem Rohling des Rohlingssortiment herausgearbeitet, der eine mit der Teilendfläche 2.1, 2.2 und 2.3 des ausgewählten Zahns 60.1, 60.2 und 60.3 aus der Zahndatenbank übereinstimmende Teilendfläche aufweist. Nach der Bearbeitung bleibt die Teilendfläche 2.1, 2.2 und 2.3 zumindest in einem zentralen Bereich unverändert. Die Lagebeziehung der zu bearbeitenden Oberflächen 6.1 bis 6.3 zueinander, zu den Teilendflächen 2.1 bis 2.3 und zu den Haltern 4.1 bis 4.3 ist bekannt. Vor dem Bearbeitungsvorgang wird ein Kalibrierungsvorgang zur Positionsbestimmung des Rohlings durchgeführt. Dabei wird die Position von mindestens drei Punkten an den verschiedenen zu bearbeitenden Oberflächen 6.1 bis 6.3, die hier Teil einer quaderförmigen Grundform sind, relativ zur Bearbeitungsmaschine bestimmt. Anschließend wird die Lagebeziehung der Teilendfläche 2.1 bis 2.3 relativ zur Bearbeitungsmaschine aus der bekannten Lagebeziehung der zu bearbeitenden Oberflächen 6.1 bis 6.3 zum Halter 4.1 bis 4.3 und des Halters 4.1 bis 4.3 zur Bearbeitungsmaschine abgeleitet.

**Bezugszeichenliste**

| | |
|---|---|
| 1 | Rohling |
| 2 | Teilendfläche |
| 3 | Transluzenzschicht |
| 4 | Halter |
| 5 | Schichten mit unterschiedlichen Farbeigenschaften |
| 6 | zu bearbeitenden Flächen |
| 7 | Teilkonstruktionsfläche |
| 8 | Lumen |
| 10 | Zahnersatzteil für einen Schneidezahn |
| 11 | Zervikalfläche |
| 12 | Inzisalkante |
| 13 | Mesialfläche |
| 14 | Distalfläche |
| 15 | Lingualfläche |
| 16 | Labialfläche |
| 20 | Rohling |
| 21 | Bukkalfläche |
| 22 | Okklusalfläche |
| 23 | Lumen |
| 24 | Fissuren |
| 30 | Zahnersatzteil für einen Backenzahn |
| 31 | Zervikalfläche |
| 32 | Mesialfläche |
| 33 | Distalfläche |
| 34 | Lingualfläche |
| 35 | Verbindungssteg |
| 40 | Rand der Teilkonstruktionsfläche |
| 41 | Transluzenzschicht |
| 50 | Computer |
| 51 | Anzeigeeinheit |
| 52 | Computertastatur |
| 53 | Computermaus |
| 54 | Datenspeicher |
| 60.1-60.3 | 3D-Modelle von Zähnen |
| 61.1-61.3 | Restflächen |
| 2.1-2.3 | Teilendflächen |
| 70.1-70.3 | 3D-Modelle von Rohlingen |
| 6.1-6.3 | zu bearbeitenden Oberflächen |
| 4.1-4.2 | Halter |
| 80.1-80.3 | Rohlinge |
| Δi | inzisaler Abstand |
| Δz | zervikaler Abstand |
| Δm | mesialer Abstand |
| Δd | distaler Abstand |

## Patentansprüche

1. Rohling zur Herstellung eines Zahnersatzteils, wobei der Rohling eine in der Geometrie und den Oberflächeneigenschaften vorgefertigte Teilendfläche des herzustellenden Zahnersatzteils aufweist, wobei der Rohling außerhalb der vorgefertigten ästhetisch relevanten Teilendfläche (2) die zu bearbeitenden Flächen (6) aufweist, die ein Übermaß gegenüber der Oberfläche (8, 12, 13, 14, 15, 31, 32, 33, 34) des bereitgestellten Zahnersatzteils (10, 30) aufweisen, **dadurch gekennzeichnet, dass** die vorgefertigte Teilendfläche (2) bei Schneidezähnen der Labialfläche (16) und vorzugsweise zusätzlich der Inzisalkante (12) bzw. bei Backenzähnen der Bukkalfläche (21) und vorzugsweise zusätzlich der Okklusalfläche (22) entspricht und wobei die zu bearbeitenden Oberflächen (6) des Rohlings Teil einer geometrischen Grundform sind, insbesondere eines Kegels, eines Zylinders, eines Würfels, eines Quaders oder einer Pyramide.

2. Rohling nach Anspruch 1, **dadurch gekennzeichnet, dass** die vorgefertigte Teilendfläche (2) höchstens 50% der gesamten Oberfläche des Rohlings ausmacht.

3. Rohling nach einem der Ansprüche 1 bis 2, **dadurch gekennzeichnet, dass** der Rohling eine Transluzenzschicht (3) aufweist und dass die vorgefertigte Teilendfläche (2) auf der Transluzenzschicht (3) angeordnet ist.

4. Rohling nach Anspruch 3, **dadurch gekennzeichnet, dass** die Transluzenzschicht (3) eine Dicke zwischen 0,3 mm und 1,5 mm aufweist und die vorzugsweise poliert ist und in ihrer Form und/oder Transluzenz und/oder Farbgebung unterschiedliche Bereiche aufweist.

5. Rohling nach einem der Ansprüche 1 bis 4, **dadurch gekennzeichnet, dass** der Rohling (1, 20) aus mindestens zwei Schichten (5) aufgebaut ist, die sich in ihrer Farbgebung und/oder in ihren Transluzenzeigenschaften voneinander unterscheiden.

6. Rohlingssortiment, umfassend mehrere Rohlinge, **dadurch gekennzeichnet, dass** das Rohlingssortiment mindestens voneinander dem Typ nach gleiche und in den Abmessungen verschiedenen Rohlinge (1, 20) nach einem der Ansprüche 1 bis 5 umfasst.

7. Verfahren zur Herstellung eines Zahnersatzteils, umfassend die Bereitstellung eines 3D-Modells eines Zahnersatzteils und die Auswahl eines geeigneten Rohlings nach einem des Ansprüche 1 bis 5 aus einer Mehrzahl von möglichen Rohlingen nach einem des Ansprüche 1 bis 5, wobei im Rahmen der Bereitstellung des 3D-Modells des Zahnersatzteils an dem 3D-Modell des Zahnersatzteils (10, 30) eine ästhetisch relevante Teilkonstruktionsfläche (7) als Teil einer äußeren Oberfläche des Zahnersatzteils (10, 30) festgelegt ist, dass aus der Mehrzahl von Rohlingen (1, 20) mit einer vorgefertigten ästhetisch relevanten Teilendfläche (2) einer Zahnoberfläche ein Rohling (1, 20) bestimmt wird, dessen Teilendfläche (2) zumindest näherungsweise der festgelegten Teilkonstruktionsfläche (7) entspricht und dass das 3D-Modell des Zahnersatzteils (10, 30) aus dem ausgewählten Rohling (1, 20) derart herausgearbeitet wird, dass die vorgefertigte Teilendfläche (2) des Rohlings (10, 30) in einem zentralen Bereich unbearbeitet bleibt wobei die festgelegte Teilkonstruktionsfläche (7) bei Schneidezähnen der Labialfläche (16) und vorzugsweise zusätzlich der Inzisalkante (12) bzw. bei Backenzähnen der Bukkalfläche (21) und vorzugsweise zusätzlich der Okklusalfläche (22) entspricht und wobei der Rohling nur im Randbereich der Teilendfläche (2) und außerhalb der Teilendfläche (2) an den zu bearbeitende Flächen (6) bearbeitet wird.

8. Verfahren nach Anspruch 7, **dadurch gekennzeichnet, dass** im Rahmen der Bereitstellung des 3D-Modells des Zahnersatzteils (10, 30) ein zu der Präparationsstelle passendes 3D-Modell eines Zahns aus einer Zahndatenbank mit einer Mehrzahl von 3D-Modellen (60.1 bis 60.3) eines Zahns mit mindestens einer festgelegten ästhetisch relevanten Teilendfläche (2.1 bis 2.3) ausgewählt wird, wobei zu jedem 3D-Modell (60.1 bis 60.3) eines Zahns aus der Zahndatenbank ein 3D-Modell (70.1 bis 70.3) eines Rohlings mit einer mit der Teilendfläche (2.1 bis 2.3) des Zahns übereinstimmenden Teilendfläche (2.1 bis 2.3) in einer Rohlingsdatenbank vorhanden ist, dass das ausgewählte 3D-Modell (60.1 bis 60.3) des Zahns zur Anpassung an die individuelle Zahnsituation der Präparationsstelle verändert wird, wobei die festgelegte Teilendfläche (2.1 bis 2.3) zumindest in einem zentralen Bereich unverändert bleibt, dass ein zu der Rohlingsdatenbank passendes Rohlingssortiment vorhanden ist und dass das 3D-Modell des Zahnersatzteils (10, 30) aus dem dem 3D-Modell des Zahns entsprechenden Rohling (80.1 bis 80.3) herausgearbeitet wird.

9. Verfahren nach einem der Ansprüche 7 bis 8, **dadurch gekennzeichnet, dass** die zu bearbeitenden Flächen (6) außerhalb der Teilendfläche (2) des Rohlings (1, 20) ein Übermaß zur Oberfläche des anzufertigenden Zahnersatzteils (10, 30) von mindestens 1 mm aufweisen.

10. Verfahren nach einem der Ansprüche 7 bis 9, **dadurch gekennzeichnet, dass** die festgelegte Teilkonstruktionsfläche (7) höchstens 50% der gesamten äußeren Oberfläche des 3D-Modells des Zahnersatzteils ausmacht.

11. Verfahren nach einem der Ansprüche 7 bis 10 **dadurch gekennzeichnet, dass** der Rohling (1, 20) aus einer Vielzahl von Rohlingen (1, 20) ausgewählt wird, die in ihrer Form und/oder Farbgebung und/oder Transluzenzeigenschaften abweichende vorgefertigte Teilendflächen (2) aufweisen.

## Claims

1. A blank for the production of a dental prosthetic item and exhibiting a prefabricated terminal subsurface corresponding in geometry and surface attributes to the planned dental prosthetic item, wherein said blank exhibits surfaces (6) to be machined which are outside the prefabricated esthetically relevant terminal subsurface (2) and are of larger dimensions than the surface (8, 12, 13, 14, 15, 31, 32, 33, 34) of the planned dental prosthetic item (10, 30), **characterized in that** the prefabricated terminal subsurface (2) corresponds, in the case of incisors, to the labial surface (16) and preferably also to the incisor edge (12) or, in the case of premolars, to the buccal surface (21) and preferably also to the occlusal surface (22), and wherein the surfaces (6) to be machined on the blank form part of a basic geometrical shape, in particular a cone, a cylinder, a cube, a cuboid, or a pyramid.

2. The blank as defined in claim 1, **characterized in that** the prefabricated terminal subsurface (2) covers not more than 50 % of the entire surface of the blank.

3. The blank as defined in claim 1 or claim 2, **characterized in that** the blank exhibits a translucent layer (3) and that the prefabricated terminal subsur-face (2) extends over the translucent layer (3).

4. The blank as defined in claim 3, **characterized in that** said translucent layer (3) has a thickness of from 0.3 mm to 1.5 mm and is preferably polished and has regions which differ in shape and/or translucency and/or color attributes.

5. The blank as defined in any one of claims 1 to 4, **characterized in that** said blank (1, 20) is composed of at least two layers (5) differing from each other as regards their color and/or translucency attributes.

6. An assortment of blanks, comprising a plurality of blanks, **characterized in that** said assortment of blanks comprises at least blanks (1, 20) of the same type but differing from each other in size as defined in any one of claims 1 to 5.

7. A method for the production of a dental prosthetic item, comprising the provision of a 3D model of a dental prosthetic item and the selection of a suitable blank as defined in any one of claims 1 to 5 from a plurality of possible blanks as defined in any one of claims 1 to 5, wherein the provision of a 3D model of the dental prosthetic item comprises the determination of an esthetically relevant designed subregion (7) of said 3D model of said dental prosthetic item (10, 30) as part of an exterior surface of said dental prosthetic item (10, 30), and a blank (1, 20) is specified from a plurality of blanks (1, 20) having a prefabricated esthetically relevant terminal subsurface (2) of a surface of the tooth, the terminal subsurface (2) of said blank being such as at least approximates the thus specified designed subregion (7), and said 3D model of said dental prosthetic item (10, 30) is carved from the selected blank (1, 20) such that the prefabricated terminal subsurface (2) of said blank (10, 30) remains unmachined in a central region thereof, and the specified designed subregion (7) corresponds, in the case of incisors, to the labial surface (16) and preferably also to the incisor edge (12), or, in the case of premolars, to the buccal surface (21) and preferably also to the occlusal surface (22) and said blank is machined only in the marginal area of said terminal subsurface (2) and on the surfaces (6) requiring machining outside the terminal subsurface (2).

8. The method as defined in claim 7, **characterized in that** the provision of said 3D model of said dental prosthetic item (10, 30) comprises the selection of a 3D model of a tooth matching the preparation site from a tooth database having a plurality of 3D models (60.1 to 60.3) of a tooth having at least one desired esthetically relevant terminal subsurface (2.1 to 2.3), wherein for each 3D model (60.1 to 60.3) of a tooth in the tooth database there is present, in a database of blanks, a 3D model (70.1 to 70.3) of a blank having a terminal subsurface (2.1 to 2.3) coinciding with said terminal subsurface (2.1 to 2.3) of said tooth and that the selected 3D model (60.1 to 60.3) of the tooth is changed for adaptation to the individual tooth situation at the preparation site, wherein said specified terminal subsurface (2.1 to 2.3) remains unchanged in at least a central region thereof, that an assortment of blanks matching the database of blanks exists, and that said 3D model of said dental prosthetic item (10, 30) is carved from said blank (80.1 to 80.3) corresponding to said 3D model of said tooth.

9. The method as defined in claim 7 or claim 8, **characterized in that** the surfaces to be machined (6) outside said terminal subsurface (2) of said blank (1, 20) exceed the size of the surface of the planned dental prosthetic item (10, 30) by at least 1 mm.

10. The method as defined in any one of claims 7 to 9, **characterized in that** said specified designed subregion (7) covers not more than 50 % of the entire exterior surface of the 3D model of said dental prosthetic item.

11. The method as defined in any one of claims 7 to 10, **characterized in that** said blank (1, 20) is selected from a large number of blanks (1, 20), which have prefabricated terminal subsurfaces (2) showing deviating shapes and/or coloration and/or translucency attributes.

## Revendications

1. Ebauche pour la fabrication d'un élément de prothèse dentaire, dans laquelle l'ébauche présente une face terminale partielle préfabriquée au niveau de la géométrie et des propriétés de surface, dans laquelle l'ébauche présente en dehors de la face terminale partielle préfabriquée (2) et appropriéesur le plan esthétique les surfaces à façonner (6) qui présentent une surcote par rapport à la surface (8, 12, 13, 14, 15, 31, 32, 33, 34) de l'élément de prothèse dentaire (10, 30) fourni, **caractérisée en ce que** la face terminale partielle préfabriquée (2) correspond pour les incisives à la face labiale (16) et de préférence de plus au bord incisal (12) ou pour les molaires à la face buccale (21) et de préférence de plus à la face occlusale (22), et dans laquelle les surfaces à façonner (6) de l'ébauche font partie d'une forme géométrique de base, en particulier d'un cône, d'un cylindre, d'un cube, d'un parallélépipède ou d'une pyramide.

2. Ebauche selon la revendication 1, **caractérisée en ce que** la face terminale partielle préfabriquée (2) occupe au maximum 50 % de la surface totale de l'ébauche.

3. Ebauche selon l'une quelconque des revendications 1 à 2, **caractérisée en ce que** l'ébauche présente une couche translucide (3) et **en ce que** la face terminale partielle préfabriquée (2) est disposée sur la couche translucide (3).

4. Ebauche selon la revendication 3, **caractérisée en ce que** la couche translucide (3) présente une épaisseur entre 0,3 mm et 1,5 mm et est de préférence polie et présente des zones différentes par leur forme et/ou leur translucidité et/ou leur coloration.

5. Ebauche selon l'une quelconque des revendications 1 à 4, **caractérisée en ce que** l'ébauche (1, 20) est constituée d'au moins deux couches (5) qui se distinguent l'une de l'autre par leur coloration et/ou par leurs propriétés de translucidité.

6. Assortiment d'ébauches comprenant plusieurs ébauches, **caractérisé en ce que** l'assortiment d'ébauches comprend au moins des ébauches (1, 20) selon l'une quelconque des revendications 1 à 5 de type identique et de dimensions différentes.

7. Procédé de fabrication d'un élément de prothèse dentaire, comprenant la fourniture d'un modèle 3D d'un élément de prothèse dentaire et la sélection d'une ébauche adéquate selon l'une quelconque des revendications 1 à 5 parmi une pluralité d'ébauches possibles selon l'une quelconque des revendications 1 à 5, dans lequel dans le cadre de la fourniture du modèle 3D de l'élément de prothèse dentaire, sur le modèle 3D de l'élément de prothèse dentaire (10, 30), une surface de construction partielle (7) appropriée sur le plan esthétique est définie comme une partie d'une surface extérieure de l'élément de prothèse dentaire (10, 30) en ce que parmi la pluralité d'ébauches (1, 20) ayant une face terminale partielle préfabriquée (2) appropriée sur le plan esthétique d'une surface dentaire, une ébauche (1, 20) est déterminée dont la face terminale partielle (2) correspond au moins approximativement à la surface de construction partielle définie (7), et en ce que le modèle 3D de l'élément de prothèse dentaire (10, 30) est élaborée à partir de l'ébauche (1, 20) sélectionnée de telle sorte que la face terminale partielle préfabriquée (2) de l'ébauche (10, 30) reste non façonnée dans une zone centrale, dans lequel la surface de construction partielle définie (7) correspond pour les incisives à la face labiale (16) et de préférence de plus au bord incisal (12) ou pour les molaires à la face buccale (21) et de préférence de plus à la face occlusale (22), et dans lequel l'ébauche est façonnée uniquement dans la zone marginale de la face terminale partielle (2) et en dehors de la face terminale partielle (2) sur les surfaces à façonner (6).

8. Procédé selon la revendication 7, **caractérisé en ce que** dans le cadre de la fourniture du modèle 3D de l'élément de prothèse dentaire (10, 30), un modèle 3D adapté au site de préparation d'une dent est sélectionné dans une base de données dentaire comprenant une pluralité de modèles 3D (60.1 à 60.3) d'une dent avec au moins une face terminale partielle (2.1 à 2.3) définie et appropriée sur le plan esthétique, dans lequel pour chaque modèle 3D (60.1 à 60.3) d'une dent de la base de données dentaire, il existe un modèle 3D (70.1 à 70.3) d'une ébauche ayant une face terminale partielle (2.1 à 2.3) coïncidant avec la face terminale partielle (2.1 à 2.3) de la dent, **en ce que** le modèle 3D sélectionné (60.1 à 60.3) de la dent est modifié pour s'adapter à la situation dentaire individuelle du site de préparation, dans lequel la face terminale partielle définie (2.1 à 2.3) reste inchangée au moins dans une zone centrale, **en ce qu'**il existe un assortiment d'ébauches assorti à la base de données d'ébauches, et **en ce que** le modèle 3D de l'élément de prothèse dentaire (10, 30) est élaboré à partir de l'ébauche (80.1 à 80.3) correspondant au modèle 3D de la dent.

9. Procédé selon l'une quelconque des revendications 7 à 8, **caractérisé en ce que** les surfaces à façonner (6) présentent en dehors de la face terminale partielle (2) de l'ébauche (1, 20) une surcote d'au moins 1 mm par rapport à la surface de l'élément de prothèse dentaire (10, 30) à réaliser.

10. Procédé selon l'une quelconque des revendications 7 à 9, **caractérisé en ce que** la surface de construction partielle définie (7) occupe au maximum 50 % de la surface extérieure totale du modèle 3D de l'élément de prothèse dentaire.

11. Procédé selon l'une quelconque des revendications 7 à 10, **caractérisé en ce que** l'ébauche (1, 20) est sélectionnée parmi une pluralité d'ébauches (1, 20) présentant des faces d'extrémité partielles (2) préfabriquées différentes par leur forme et/ou leur coloration et/ou leurs propriétés de translucidité.
